# FASCICULE DE BREVET EUROPEEN

(11) **EP 4 340 774 B1**
(45) Date de publication et mention de la délivrance du brevet: **24.09.2025**
(21) Numéro de dépôt: 22727371.1
(22) Date de dépôt: 09.05.2022
(51) Int. Cl.: A61D 7/00, A61M 5/20

(54) **DISPOSITIF PORTATIF D'INJECTION D'UNE SUBSTANCE PERFECTIONNE**
VERBESSERTE TRAGBARE INJEKTIONSVORRICHTUNG FÜR EINE SUBSTANZ
IMPROVED PORTABLE DEVICE FOR INJECTING A SUBSTANCE

(30) Priorité: 18.05.2021 EP 21305652
(43) Date de publication de la demande: 27.03.2024
(73) Titulaire: Desvac, 49124 Saint Barthelemy D'Anjou (FR)
(72) Inventeur: MARS, Julie, 49124 Saint Barthelemy D'Anjou (FR); L'HARIDON, Devan, 49124 Saint Barthelemy D'Anjou (FR); SALAUN, Damien, 49124 Saint Barthelemy D'Anjou (FR)
(74) Mandataire: Plasseraud IP
(86) Numéro de dépôt international: PCT/FR2022/050878
(87) Numéro de publication internationale: WO 2022/243620

(56) Documents cités:
- WO-A1-2016/181091
- KR-B1- 102 037 471
- US-A1- 2016 271 326

## Description

### Domaine technique

Le domaine de la présente invention est celui des dispositifs d'administration de substance à un ou plusieurs animaux, tels que des pistolets doseurs.

Elle concerne plus particulièrement un dispositif d'injection portatif dans lequel le système de déplacement de son aiguille rétractable et le système pour décharger une dose de substance au travers de cette aiguille sont séparés, le dispositif d'injection étant également plus compact et plus léger.

Elle concerne aussi un procédé de gestion d'un tel dispositif d'injection permettant de traiter plus rapidement un groupe entier d'animaux en réduisant le temps mort nécessaire au réarmement de ce dispositif d'injection entre deux injections successives.

### Technique antérieure

Il est connu dans le domaine de la production animale d'administrer des substances telles que des médicaments ou des vaccins à des groupes d'animaux pour les maintenir en bonne santé.

Le traitement ou la vaccination des animaux tels que du bétail, contre différentes maladies permet non seulement d'améliorer la santé de ces animaux et d'augmenter la rentabilité d'un troupeau d'animaux mais est également un facteur essentiel pour la production de denrées alimentaires d'origine animale.

Il est connu d'administrer une telle substance au moyen d'un pistolet d'injection afin de faciliter le traitement ou la vaccination d'un groupe entier d'animaux.

Divers pistolets d'injection existent ainsi dans l'état de l'art.

La demande de brevet WO2016/108187 A1 au nom de la présente demanderesse, propose un dispositif d'injection portatif comprenant un boîtier avec une poignée et une aiguille rétractable logée dans le boîtier.

Ce dispositif d'injection portatif offre une sécurité renforcée pour l'opérateur en ce qu'il comprend un premier capteur pour détecter la préhension de la poignée du dispositif par un utilisateur et un second capteur pour détecter la présence d'un animal.

Ce dispositif d'injection comporte en outre un circuit de traitement configuré pour recevoir un premier signal depuis le premier capteur indiquant que l'utilisateur tient la poignée et recevoir un deuxième signal depuis le deuxième capteur indiquant qu'un animal est détecté. Le dispositif d'injection déploie l'aiguille hors du boitier dans l'animal uniquement lorsque ces deux signaux sont reçus et délivre une dose de médicament dans l'animal une fois que l'aiguille est totalement déployée dans l'animal.

Bien que donnant de bons résultats, ce dispositif d'injection peut encore être amélioré.

En effet, le déplacement de l'aiguille hors et dans la coque du dispositif d'injection d'une part et la décharge d'une dose de substance à administrer au travers de cette aiguille d'autre part sont assurés par une seule ligne cinématique comprenant un unique moteur électrique.

Des pièces mécaniques complémentaires telles que des poussoirs à ressort, sont alors nécessaires pour différencier deux mouvements, chacun de ces mouvements étant lié à une fonction distincte : le déplacement de l'aiguille et le déplacement du piston pour décharger le contenu de la chambre.

Or, après chaque administration d'une substance, il est nécessaire que le moteur réalise une course complète jusqu'à l'arrière de la seringue pour bien réamorcer les poussoirs à ressort nécessaires à la sortie de l'aiguille.

Par ailleurs, le rechargement du dispositif d'injection avec une nouvelle dose de substance est réalisé seulement après réamorçage des poussoirs et arrêt du moteur.

Il en résulte un temps de réarmement du dispositif d'injection après administration d'une substance dans un animal, qui est relativement long et est à l'origine d'un ralentissement notable des opérations de traitement d'un groupe entier d'animaux.

En outre, il est connu que les dispositifs d'injection de l'état de l'art sont pesants et sont donc susceptibles d'entraîner une fatigue accrue de l'opérateur lorsqu'il doit traiter un grand nombre d'animaux, notamment parce qu'il doit pouvoir se servir d'un tel dispositif d'injection d'une seule main.

Des dispositifs d'injections sont également connus des documents US2016/271326 A1, WO2016/181091 Al et KR102037471 B1.

Il existe donc un besoin pressant pour un dispositif d'administration d'un produit vétérinaire à un animal tel qu'un porcelet, dont la conception originale permette de surmonter les inconvénients de l'art antérieur exposés ci-dessus.

### Objet de l'invention

La présente invention vise à pallier les inconvénients de l'art antérieur en proposant un dispositif portatif d'injection d'une substance, simple dans sa conception et dans son mode opératoire, dont le temps de réarmement, ou d'indisponibilité, après administration d'une substance à un animal est sensiblement réduit, autorisant en conséquence un traitement plus rapide d'un groupe entier d'animaux.

Un autre objet de la présente invention est un tel dispositif d'injection d'une substance présentant un poids sensiblement diminué.

Un autre objet de la présente invention est un tel dispositif d'injection d'une substance moins encombrant, avec un centre de gravité bien positionné pour donner l'impression à l'utilisateur d'une prise en main aisée.

La présente invention concerne également un procédé de gestion d'un tel dispositif d'injection permettant de réduire le temps de réarmement, ou d'indisponibilité, de ce dispositif d'injection entre deux administrations successives d'une substance.

### Exposé de l'invention

A cet effet, l'invention concerne un dispositif portatif d'injection d'une substance tel que défini dans la revendication 1 et un procédé de gestion d'un dispositif d'injection d'une substance tel que défini dans la revendication 13. Des modes de réalisation sont définis dans les revendications dépendantes. Le dispositif portatif d'injection d'une substance comprend un circuit d'alimentation comportant une chambre comprenant une première extrémité avec un orifice de sortie et une seconde extrémité ouverte, un piston s'étendant vers l'intérieur de ladite chambre à partir de cette seconde extrémité, ladite chambre présentant un volume variable par déplacement du piston, un premier moteur électrique pour assurer le déplacement dudit piston, ledit orifice de sortie de ladite chambre étant relié à une aiguille d'injection rétractable pour alimenter cette aiguille avec le contenu de ladite chambre.
Selon l'invention,
- ledit dispositif d'injection comportant un corps de dispositif d'injection, ledit circuit d'alimentation est placé dans ledit corps de dispositif d'injection portatif, et
- le corps de dispositif d'injection comprend un système d'entraînement de ladite aiguille entre une première position, dans laquelle l'aiguille est logée à l'intérieur du corps du dispositif d'injection, et une deuxième position, dans laquelle l'aiguille fait saillie hors du corps du dispositif d'injection, ledit système d'entraînement de ladite aiguille comprenant un deuxième moteur distinct dudit premier moteur électrique de sorte que les fonctions d'entrée/sortie de l'aiguille d'une part et de réamorçage/délivrance, ou recharge/décharge, d'une dose de substance, d'autre part, sont dissociées.

Un tel dispositif d'injection permet avantageusement de dissocier le système d'entraînement de l'aiguille rétractable et le système mécanique permettant de décharger une dose de substance au travers de cette aiguille.

Ce dispositif d'injection portatif peut être mis en œuvre pour administrer une substance à du bétail, notamment des porcs, des moutons, des veaux, des bovins, des chèvres, etc... mais également des chiens et des chats.

Selon l'invention telle que revendiquée le dispositif portatif d'injection d'une substance comporte des moyens pour synchroniser les premier et deuxième moteurs électriques de sorte que la rentrée de l'aiguille après injection de ladite substance et le réamorçage de la chambre sont réalisés simultanément, ou encore en parallèle.
Par exemple, ledit dispositif portatif peut comporter une unité de commande électronique apte à commander chacun des premier et deuxième moteurs, ladite unité de commande électronique étant configurée pour synchroniser les premier et deuxième moteurs électriques.
Il en résulte un gain de temps dans le traitement des animaux vivants significatif. Des tests ont par exemple montré pour une dose de substance de 2ml, un gain de temps de 490 ms sur le cycle complet.

Selon un autre mode de réalisation de ce dispositif portatif d'injection d'une substance, le premier moteur électrique est un moteur pas à pas.
Afin d'entrainer ledit piston dans un mouvement de translation à l'intérieur de la chambre, ce moteur pas à pas entraîne en rotation un système de transformation de mouvement rotation/translation.
Ce système de transformation de mouvement rotation/translation est de préférence un système vis/écrou à billes.
Alternativement, il pourrait encore s'agir d'un système comprenant des pignons et une courroie ou encore d'un système pignon/crémaillère.
Le système vis/écrou à billes est préféré en raison de son poids moindre, lequel peut représenter un allègement pouvant atteindre jusqu'à quarante pour cent (40%), et préférentiellement trente-quatre pour cent (34%) par rapport au poids d'autres systèmes d'entraînement connus.

Selon encore un autre mode de réalisation de ce dispositif portatif d'injection d'une substance, ledit système d'entraînement de ladite aiguille comprend un organe d'entrainement déplacé par un servomoteur.
La mise en œuvre d'un servomoteur permet avantageusement de ne pas alourdir le dispositif d'injection, le poids de ce servomoteur étant, à titre purement illustratif, de l'ordre de seulement plusieurs dizaines de grammes.
De manière avantageuse, cet organe d'entrainement est un système bielle/manivelle. Alternativement, il pourrait encore s'agir d'une liaison pivot. Le servomoteur permet de déplacer la bielle en rotation, cette dernière déplaçant alors l'aiguille d'injection en translation. Cette configuration permet avantageusement de limiter l'encombrement du système d'entraînement de l'aiguille d'injection.

Selon encore un autre mode de réalisation de ce dispositif portatif d'injection d'une substance, cette aiguille d'injection étant portée par ledit circuit d'alimentation, ledit organe d'entrainement est relié audit circuit d'alimentation de sorte que, lorsque ledit organe d'entrainement est déplacé par ledit servomoteur, ledit circuit d'alimentation est déplacé dans le corps de dispositif d'injection pour entraîner en mouvement ladite aiguille entre ses première et deuxième positions.
Ainsi le déplacement de l'aiguille entre sa première position et sa deuxième position est assuré par le déplacement du circuit d'alimentation tout entier.
De préférence, l'organe d'entrainement est solidaire du premier moteur pour entraîner ce dernier en déplacement.
De manière avantageuse, cet organe d'entrainement et ce servomoteur sont placés sous ce circuit d'alimentation dans le corps de dispositif d'injection.

Selon encore un autre mode de réalisation de ce dispositif portatif d'injection d'une substance, et de manière alternative au mode de réalisation précédent, ladite aiguille étant portée par un support d'aiguille distinct dudit circuit d'alimentation, ledit organe d'entrainement est relié audit support d'aiguille pour déplacer ladite aiguille entre ses première et deuxième positions. Le circuit d'alimentation et l'ensemble comprenant ladite aiguille et son système d'entrainement propre sont alors distincts en étant espacés l'un de l'autre dans le corps de dispositif d'injection.
Seule l'aiguille d'injection rétractable est reliée à l'orifice de sortie de la chambre par une liaison souple telle qu'une tubulure, pour recevoir le contenu de cette chambre lors de son déchargement.

Selon encore un autre mode de réalisation de ce dispositif portatif d'injection d'une substance, il comprend une poignée de préhension.
Cette poignée de préhension est éventuellement inclinée, ou forme un angle supérieur ou égal à 90°, par rapport à l'axe longitudinal de la partie de corps principale du dispositif d'injection dans laquelle l'aiguille est logée et le long duquel cette aiguille se déplace.

Selon encore un autre mode de réalisation de ce dispositif portatif d'injection d'une substance, ce dispositif d'injection comprenant une poignée de préhension, le circuit d'alimentation est logé au moins en partie dans cette poignée de préhension.
De manière plus générale, au moins une portion du circuit d'alimentation, et notamment sa chambre, est agencée dans le dispositif portatif d'injection d'une substance de manière non horizontale lorsque la partie de corps principal est tenue parallèlement à la surface du sol.
De manière avantageuse, une telle configuration facilite l'évacuation d'une éventuelle bulle d'air aspirée dans le circuit d'alimentation.
En outre, le centre de gravité du dispositif d'injection portatif est positionné juste au-dessus du poignet de l'opérateur, donnant l'impression à ce dernier que le dispositif d'injection est aisé à tenir.

Selon encore un autre mode de réalisation de ce dispositif portatif d'injection d'une substance, il comporte un module électronique configuré pour ajuster le signal d'alimentation de la bobine du premier moteur en fonction du couple résistant s'appliquant au rotor de ce premier moteur électrique. L'ajustement du signal d'alimentation, d'une fréquence donnée et d'une forme d'onde donnée, de la bobine du premier moteur électrique pour déplacer le piston dans la chambre afin de décharger cette dernière, est donc automatique dès qu'une nouvelle substance à administrer est introduite dans la chambre. A titre d'exemple, ce signal d'alimentation est une onde sinusoïdale.

Selon encore un autre mode de réalisation de ce dispositif portatif d'injection d'une substance, ladite chambre est dimensionnée pour déterminer une dose d'injection à administrer à un animal.
A titre purement illustratif, cette chambre est un corps cylindrique creux.
De préférence, le volume de ladite chambre est compris entre zéro (0) et dix (10) ml, de préférence entre zéro (0) et cinq (5) ml.
A titre d'exemple, le volume de cette chambre est de trois (3) ml.

Selon encore un autre mode de réalisation de ce dispositif portatif d'injection d'une substance, il comprend un ou plusieurs éléments choisis parmi :
- un capteur de mouvement tel qu'un accéléromètre, un inclinomètre, un gyromètre,
- un capteur de température,
- un capteur d'humidité,
- un capteur temporel tel qu'une horloge, un calendrier, ...,
- un capteur de position tel qu'un dispositif GPS/GALILEO, un dispositif de géolocalisation, balise, ...
- un dispositif de communication sans contact, et/ou
- un module de communication sans fil pour communiquer avec un réseau.
De préférence, ce dispositif portatif est un équipement connecté à un réseau pour transmettre et/ou recevoir des données.
Les communications peuvent être réalisées en utilisant un réseau local tel que Wifi, Lifi, Ethernet, ... ou un réseau d'accès cellulaire, lequel peut être de plusieurs types (2G, 3G, 4G, 5G), chaque type de réseau étant accessible selon plusieurs technologies d'accès cellulaires (2G : EDGE, GPRS, 3G : UMTS, HSDPA, HSUPA, HSPA, HSPA+, 4G : LTE, 5G).
Alternativement, il peut encore s'agir d'un module de communication sans fil à faible consommation énergétique, tel qu'un module configuré pour communiquer selon un des protocoles suivants : Bluetooth, Sigfox, LoRa, THREAD, Wifi Halow, ou encore ZigBee.
L'accéléromètre peut être mis en œuvre pour mesurer l'angle d'inclinaison du dispositif portatif lors de l'injection de ladite substance dans l'animal. Le capteur de température peut servir à mesurer la température de cette substance lors de son injection.
Le dispositif de communication sans contact peut être un lecteur RFID (radiofréquence) ou un lecteur NFC ("Near Field Communication" - communication en champ proche), notamment utilisé pour identifier l'animal porteur d'une étiquette ou d'un tag d'identification individuelle.
L'ensemble de ces informations collectées issues de différentes sources peuvent permettre d'assurer un suivi et une notation de la qualité de chaque injection réalisée.
Ces informations peuvent encore être utilisées pour déterminer le nombre d'injections réalisées avec un réservoir tel qu'un flacon alimentant la chambre avec ladite substance. Lorsqu'une valeur seuil prédéterminée est atteinte, un signal d'alarme peut être adressé à l'opérateur pour prévenir du changement requis du réservoir

Ce dispositif portatif d'injection d'une substance peut également comporter un ou plusieurs capteurs de pression pour déterminer par exemple un contact avec l'animal afin de déclencher la sortie de l'aiguille et l'injection

Ce dispositif portatif d'injection d'une substance peut encore comporter une sonde pour mesurer le niveau et l'utilisation de sa batterie.

Selon encore un autre mode de réalisation de ce dispositif portatif d'injection d'une substance, il comprend une unité principale apte à commander lesdits premier et deuxième moteurs, ladite unité principale comportant un processeur et un ensemble d'instructions logicielles qui lorsqu'elles sont exécutées par ledit processeur, permettent de mettre en œuvre un procédé de gestion d'un dispositif d'injection d'une substance, dans lequel ladite aiguille étant dans sa deuxième position et le contenu de ladite chambre ayant été déchargé, on remplit ladite chambre avec ladite substance pendant le déplacement de ladite aiguille de sa deuxième position vers sa première position.

Bien entendu, l'expression "processeur" s'entend de tout dispositif programmable.

La présente invention concerne également un procédé de gestion d'un dispositif d'injection d'une substance comprenant un circuit d'alimentation comportant une chambre comprenant une première extrémité avec un orifice de sortie et une seconde extrémité ouverte, un piston s'étendant vers l'intérieur de ladite chambre à partir de cette seconde extrémité, ladite chambre présentant un volume variable par déplacement du piston, un premier moteur électrique pour assurer le déplacement dudit piston, ledit orifice de sortie de ladite chambre étant relié à une aiguille d'injection rétractable pour alimenter cette aiguille avec le contenu de ladite chambre.
Selon l'invention,
- le corps de dispositif d'injection comprenant un système d'entraînement de ladite aiguille entre une première position, dans laquelle l'aiguille est logée à l'intérieur du corps du dispositif d'injection, et une deuxième position, dans laquelle l'aiguille fait saillie hors du corps du dispositif d'injection, ledit système d'entraînement de ladite aiguille comprenant un deuxième moteur distinct dudit premier moteur électrique de sorte que les fonctions d'entrée/sortie de l'aiguille et de réamorçage/délivrance, ou recharge/décharge, d'une dose de substance sont dissociées,
- on remplit ladite chambre avec ladite substance pendant le déplacement de ladite aiguille de sa deuxième position vers sa première position.

De préférence, ladite chambre est entièrement remplie avec ladite substance pour déterminer une nouvelle dose à injecter au plus tard lorsque ladite aiguille arrive dans sa première position.

La présente divulgation concerne également un programme d'ordinateur comprenant des instructions adaptées à la mise en œuvre de chacune des étapes du procédé de gestion d'un dispositif d'injection d'une substance, tel que décrit précédemment, lorsque ledit programme est exécuté sur un ordinateur.

Un tel programme d'ordinateur est téléchargeable depuis un réseau de communication et/ou enregistré sur un support lisible par ordinateur et/ou est exécutable par un processeur.

La présente divulgation concerne encore l'utilisation d'un dispositif d'injection d'une substance tel que décrit précédemment pour l'administration à des animaux de substances actives telles que des produits vétérinaires, des préparations non thérapeutiques, des vitamines, des vaccins, ...

### Brève description des dessins

D'autres avantages, buts et caractéristiques particulières de la présente invention ressortiront de la description qui va suivre, faite, dans un but explicatif et nullement limitatif, en regard des dessins annexés, dans lesquels :
**Fig. 1**

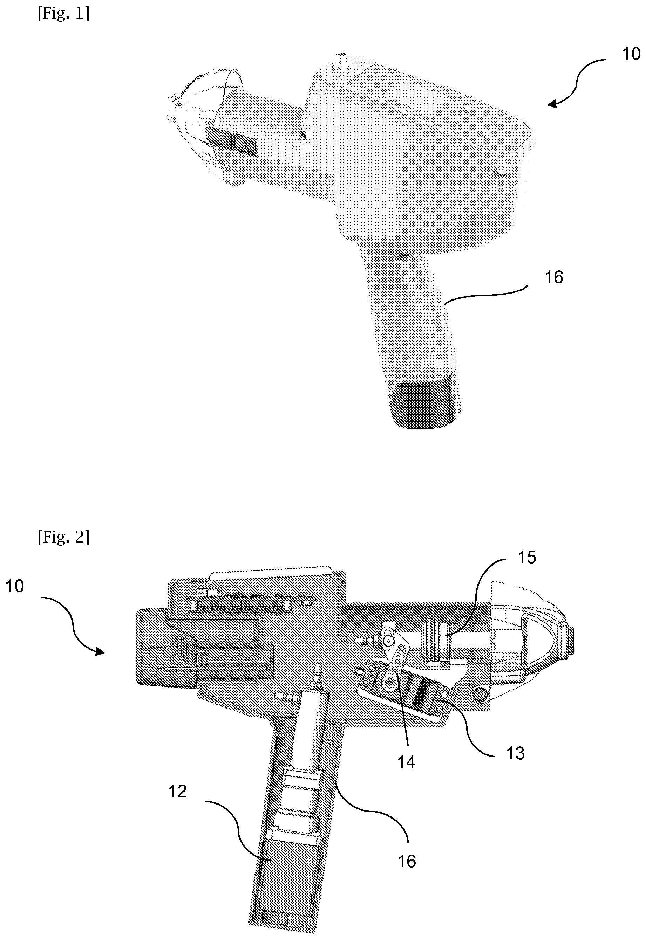

   [Fig. 1] est une vue en perspective d'un dispositif d'injection d'une substance selon un premier mode de réalisation particulier de la présente invention ;
**Fig. 2**
   [Fig. 2] est une vue en coupe du dispositif d'injection de la Fig. 1 montrant l'agencement du système d'entrainement de l'aiguille d'injection et celui du circuit d'alimentation de ladite aiguille dans le corps de dispositif d'injection ;
**Fig. 3**

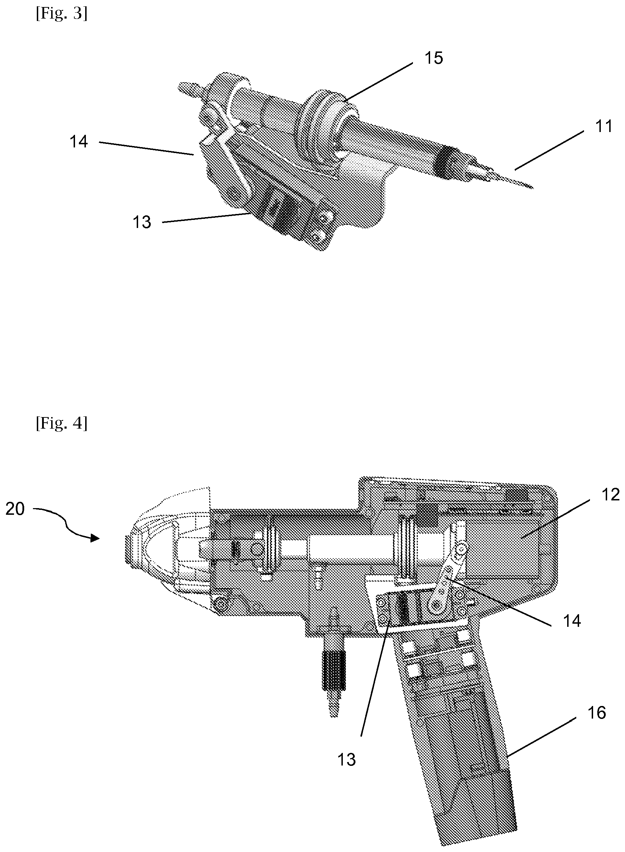

   [Fig. 3] est une vue en perspective du système d'entrainement de l'aiguille d'injection du dispositif d'injection de la Fig. 1 ;
**Fig. 4**
   [Fig. 4] est une vue en coupe d'un dispositif d'injection selon un second mode de réalisation de la présente invention, l'agencement du système d'entrainement de l'aiguille d'injection et l'agencement du circuit d'alimentation de ladite aiguille dans le corps de dispositif d'injection étant visibles ;
**Fig. 5**

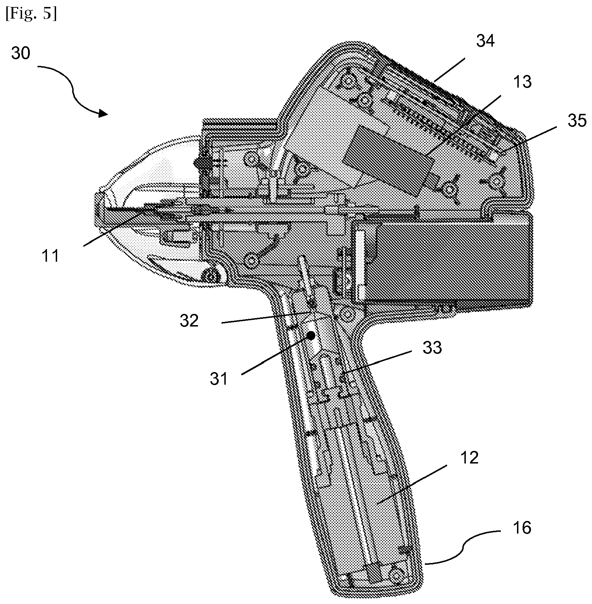

   [Fig. 5] est une vue en coupe d'un dispositif d'injection selon un troisième mode de réalisation de la présente invention ;

### Description des modes de réalisation

Les dessins et la description ci-après contiennent, pour l'essentiel, des éléments de caractère certain. Ils pourront donc non seulement servir à mieux faire comprendre la présente invention, mais aussi contribuer à sa définition, le cas échéant.

Tout d'abord, on note que les figures ne sont pas à l'échelle.

Les Figures 1 à 3 illustrent de manière schématique un dispositif portatif d'injection d'une substance selon un premier mode de réalisation de la présente invention.

Ce dispositif d'injection 10 comporte une aiguille rétractable 11 pour l'injection d'une substance dans un animal, ici un porcelet. De manière connue, cette aiguille d'injection 11 est une aiguille hypodermique réalisée en acier inoxydable.

Ce dispositif d'injection 10 comporte un circuit d'alimentation en fluide pour décharger au travers de cette aiguille d'injection 11 une dose d'une substance à administrer.

Ce circuit d'alimentation (partiellement représenté) comporte une chambre qui présente ici un corps cylindrique creux, laquelle comprend une première extrémité avec un orifice de sortie débouchant sur une tubulure en communication de fluide avec le canal intérieur de l'aiguille d'injection 11 et une seconde extrémité ouverte, un piston s'étendant vers l'intérieur de cette chambre à partir de cette seconde extrémité.

Le pourtour de la tête du piston qui est également cylindrique, assure avec la paroi intérieure de la chambre, une étanchéité au fluide. Cette chambre présente ainsi un volume variable par le déplacement du piston dans celle-ci.

Le volume maximal de cette chambre définit une dose de substance à administrer au porcelet, telle qu'une dose de 3 ml.

Pour assurer le déplacement de ce piston, à l'intérieur de la chambre, le dispositif d'injection comporte un moteur pas à pas 12. Ce moteur pas à pas 12 entraîne en rotation un système de transformation de mouvement rotation/translation qui est ici un système vis/écrou à billes.

Ainsi cette unité motrice comprenant le moteur pas à pas 12 et le système vis/écrou à billes permet de déplacer en translation le piston à l'intérieur de la chambre pour faire varier son volume et décharger son contenu au travers de l'aiguille d'injection 11.

Le système d'entrainement de l'aiguille d'injection 11 comprend pour sa part, un servomoteur 13, ce dernier développant assez de couple pour piquer l'aiguille 11 dans l'échine du porcelet.

Le système d'entrainement de l'aiguille d'injection 11 et le système pour décharger une dose de substance au travers de cette aiguille d'injection 11 comprennent donc chacun un moteur propre.

Pour déplacer l'aiguille d'injection 11 entre sa première position et sa deuxième position, le servomoteur 13 déplace en rotation un organe d'entrainement qui est ici un système bielle/manivelle 14. Un palier 15 permet de garantir le déplacement linéaire de l'aiguille d'injection 11 entre ses deux positions extrêmes.

Le circuit d'alimentation comprenant le premier moteur électrique 12, le système vis/écrou, le piston et la chambre, est ici avantageusement logé dans la poignée de préhension 16 du dispositif d'injection 10.

Un avantage immédiat de cet agencement est que le circuit d'alimentation étant disposé dans la coque du dispositif d'injection 10 de manière non horizontale mais avec sa chambre dirigée vers le haut, il est plus facile de chasser d'éventuelles bulles d'air qui auraient été aspirées dans le circuit d'alimentation.

De plus, une telle configuration autorise une plus grande compacité du dispositif d'injection 10 tout en assurant une meilleure prise en main pour l'utilisateur, le centre de gravité du système étant mieux positionné.

Ainsi, on observe que la conception originale du dispositif d'injection illustré aux Figures 1 à 3, assure une plus grande compacité et un poids réduit à ce dernier, ce qui entraîne une fatigue moindre pour l'opérateur lors de la réalisation de ses opérations.

La figure 4 est une vue en coupe d'un dispositif portatif d'injection d'une substance selon un second mode de réalisation de la présente invention.

Les éléments portant les mêmes références que ceux représentés sur les figures 1 à 3 représentent les mêmes objets, lesquels ne seront pas décrits de nouveau ci-après.

Le dispositif portatif d'injection 20 de la figure 4 diffère de celui représenté aux figures 1 à 3 en ce que le servomoteur 13 et l'ensemble bielle/manivelle 14 sont agencés dans le corps principal de ce dispositif portatif sous le circuit d'alimentation. L'ensemble bielle/manivelle 14 relie ainsi le servomoteur 13 au premier moteur électrique 12 de sorte que le déplacement en rotation de la bielle par le servomoteur 13 entraîne un déplacement en translation du premier moteur électrique 12 à l'intérieur de l'espace délimité par la coque du dispositif d'injection 20.

Le déplacement en translation du premier moteur électrique 12 auquel est relié le reste du circuit d'alimentation de l'aiguille d'injection, ce circuit portant également l'aiguille d'injection 11, permet de réaliser la sortie et l'entrée de cette aiguille d'injection hors de et dans la coque du dispositif portatif d'injection 20.

La figure 5 est une vue en coupe d'un dispositif portatif d'injection 30 selon un troisième mode de réalisation de la présente invention.

Les éléments portant les mêmes références que ceux représentés sur les figures 1 à 3 représentent les mêmes objets, lesquels ne seront pas décrits de nouveau ci-après.

Ce dispositif d'injection 30 comporte un circuit d'alimentation en fluide pour décharger au travers de son aiguille d'injection 11 une substance à injecter.

Ce circuit d'alimentation (partiellement représenté) comporte une chambre 31 qui présente ici un corps cylindrique creux, laquelle comprend une première extrémité avec un orifice de sortie 32 débouchant sur une tubulure (non représentée) en communication de fluide avec le canal intérieur de l'aiguille d'injection 11 et une seconde extrémité ouverte, un piston 33 s'étendant vers l'intérieur de cette chambre 31 à partir de cette seconde extrémité.

Ce dispositif portatif d'injection 30 comporte également un écran d'affichage 34 permettant à l'opérateur de sélectionner des fonctions dans un menu. Un module électronique 35 pourvu d'un processeur permet de gérer l'affichage et le logiciel permettant d'opérer le dispositif d'injection 30.

## Revendications

1. Dispositif portatif d'injection d'une substance comprenant un circuit d'alimentation comportant une chambre comprenant une première extrémité avec un orifice de sortie et une seconde extrémité ouverte, un piston s'étendant vers l'intérieur de ladite chambre à partir de cette seconde extrémité, ladite chambre présentant un volume variable par déplacement du piston, un premier moteur électrique (12) pour assurer le déplacement dudit piston, ledit orifice de sortie de ladite chambre étant relié à une aiguille d'injection (11) rétractable pour alimenter cette aiguille avec le contenu de ladite chambre, ledit dispositif d'injection comportant un corps de dispositif d'injection, ledit circuit d'alimentation est placé dans ledit corps de dispositif d'injection portatif, le corps de dispositif d'injection comprenant un système d'entraînement de ladite aiguille (11) entre une première position, dans laquelle l'aiguille est logée à l'intérieur du corps du dispositif d'injection, et une deuxième position, dans laquelle l'aiguille (11) fait saillie hors du corps du dispositif d'injection, ledit système d'entraînement de ladite aiguille d'injection (11) comprenant un deuxième moteur (13) distinct dudit premier moteur électrique (12) de sorte que les fonctions d'entrée/sortie de l'aiguille d'injection (11) et de réamorçage/délivrance, ou recharge/décharge, d'une dose de substance sont dissociées, et **caractérisé en ce que** ledit dispositif d'injection comporte des moyens pour synchroniser les premier et deuxième moteurs électriques de sorte que la rentrée de l'aiguille après injection de ladite substance et le réamorçage de la chambre sont réalisés simultanément.

2. Dispositif portatif d'injection d'une substance selon la revendication 1, **caractérisé en ce que** le premier moteur électrique (12) est un moteur pas à pas.

3. Dispositif portatif d'injection d'une substance selon la revendication 1 ou 2, **caractérisé en ce que** ledit système d'entraînement de ladite aiguille d'injection (11) comprend un organe d'entrainement déplacé par un servomoteur (13).

4. Dispositif portatif d'injection d'une substance selon la revendication 3, **caractérisé en ce que** ledit organe d'entrainement est un système bielle/manivelle (14).

5. Dispositif portatif d'injection d'une substance selon la revendication 3 ou 4, **caractérisé en ce que** ladite aiguille d'injection (11) étant portée par ledit circuit d'alimentation, ledit organe d'entrainement est relié audit circuit d'alimentation de sorte que, lorsque ledit organe d'entrainement est déplacé par ledit servomoteur (13), ledit circuit d'alimentation est déplacé dans le corps de dispositif d'injection pour entraîner en mouvement ladite aiguille entre ses première et deuxième positions.

6. Dispositif portatif d'injection d'une substance selon la revendication 5, **caractérisé en ce que** ledit organe d'entrainement est solidaire dudit premier moteur (12).

7. Dispositif portatif d'injection d'une substance selon la revendication 3 ou 4, **caractérisé en ce que** ladite aiguille d'injection (11) étant portée par un support d'aiguille distinct dudit circuit d'alimentation, ledit organe d'entrainement est relié audit support d'aiguille pour déplacer ladite aiguille entre ses première et deuxième positions.

8. Dispositif portatif d'injection d'une substance selon l'une quelconque des revendications précédentes, **caractérisé en ce qu'**il comprend une poignée de préhension (16).

9. Dispositif portatif d'injection d'une substance selon l'une quelconque des revendications précédentes, **caractérisé en ce que** au moins une portion du circuit d'alimentation, et notamment sa chambre, est agencée dans le dispositif portatif d'injection de manière non horizontale lorsque la partie de corps principal est tenue parallèlement à la surface du sol.

10. Dispositif portatif d'injection d'une substance selon l'une quelconque des revendications précédentes, **caractérisé en ce qu'**il comporte un module électronique configuré pour ajuster le signal d'alimentation de la bobine du premier moteur en fonction du couple résistant s'appliquant au rotor de ce premier moteur électrique (12).

11. Dispositif portatif d'injection d'une substance selon l'une quelconque des revendications précédentes, **caractérisé en ce que** ladite chambre est dimensionnée pour déterminer une dose d'injection.

12. Dispositif portatif d'injection d'une substance selon l'une quelconque des revendications précédentes, **caractérisé en ce qu'**il comprend un ou plusieurs éléments choisis parmi un capteur de mouvement, un capteur de température, un capteur d'humidité, un capteur temporel, un capteur de position, un dispositif de communication sans contact et/ou un module de communication sans fil pour communiquer avec un réseau.

13. Procédé de gestion d'un dispositif d'injection d'une substance comprenant un circuit d'alimentation comportant une chambre comprenant une première extrémité avec un orifice de sortie et une seconde extrémité ouverte, un piston s'étendant vers l'intérieur de ladite chambre à partir de cette seconde extrémité, ladite chambre présentant un volume variable par déplacement du piston, un premier moteur électrique (12) pour assurer le déplacement dudit piston, ledit orifice de sortie de ladite chambre étant relié à une aiguille d'injection (11) rétractable pour alimenter cette aiguille avec le contenu de ladite chambre, le corps de dispositif d'injection comprenant un système d'entraînement de ladite aiguille (11) entre une première position, dans laquelle l'aiguille est logée à l'intérieur du corps du dispositif d'injection, et une deuxième position, dans laquelle l'aiguille (11) fait saillie hors du corps du dispositif d'injection, ledit système d'entraînement de ladite aiguille d'injection (11) comprenant un deuxième moteur (13) distinct dudit premier moteur électrique (12) de sorte que les fonctions d'entrée/sortie de l'aiguille d'injection (11) et de réamorçage/délivrance, ou recharge/décharge, d'une dose de substance sont dissociées, caratérisé en ce qu' en qu' - on remplit ladite chambre avec ladite substance pendant le déplacement de ladite aiguille d'injection (11) de sa deuxième position vers sa première position.

14. Procédé de gestion d'un dispositif d'injection d'une substance selon la revendication 13, **caractérisé en ce que** ladite chambre est entièrement remplie avec ladite substance pour déterminer une dose à injecter au plus tard lorsque ladite aiguille d'injection (11) arrive dans sa première position.

## Patentansprüche

1. Tragbare Vorrichtung zum Injizieren einer Substanz, umfassend einen Einspeisekreislauf mit einer Kammer, die ein erstes Ende mit einer Austrittsöffnung und ein zweites offenes Ende umfasst, einen Kolben, der sich von diesem zweiten Ende aus zum Inneren der Kammer erstreckt, wobei die Kammer aufgrund der Bewegung des Kolbens ein variables Volumen aufweist, einen ersten Elektromotor (12) zum Sicherstellen der Bewegung des Kolbens, wobei die Austrittsöffnung der Kammer zum Einspeisen des Inhalts der Kammer in diese Nadel mit einer zurückziehbaren Injektionsnadel (11) verbunden ist, wobei die Injektionsvorrichtung einen Injektionsvorrichtungskörper umfasst, wobei der Einspeisekreislauf in dem tragbaren Injektionsvorrichtungskörper angeordnet ist, wobei der Injektionsvorrichtungskörper ein Antriebssystem zum Antreiben der Nadel (11) zwischen einer ersten Position, in der die Nadel innerhalb des Injektionsvorrichtungskörpers aufgenommen ist, und einer zweiten Position, in der die Nadel (11) aus dem Injektionsvorrichtungskörper herausragt, umfasst, wobei das Antriebssystem der Injektionsnadel (11) einen zweiten Motor (13) umfasst, der sich derart von dem ersten Elektromotor (12) unterscheidet, dass die Funktionen des Eintritts/Austritts der Injektionsnadel (11) und des Wiederauffüllens/Abgebens oder des Nachfüllens/Entleerens einer Substanzdosis voneinander getrennt sind, und **dadurch gekennzeichnet, dass** die Injektionsvorrichtung Mittel zum Synchronisieren des ersten und des zweiten Elektromotors derart umfasst, dass das Zurückziehen der Nadel nach der Injektion der Substanz und das Wiederauffüllen der Kammer gleichzeitig ausgeführt werden.

2. Tragbare Vorrichtung zum Injizieren einer Substanz nach Anspruch 1,
**dadurch gekennzeichnet, dass** der erste Elektromotor (12) ein Schrittmotor ist.

3. Tragbare Vorrichtung zum Injizieren einer Substanz nach Anspruch 1 oder 2, **dadurch gekennzeichnet, dass** das Antriebssystem der Injektionsnadel (11) ein Antriebselement umfasst, das durch einen Servomotor (13) bewegt wird.

4. Tragbare Vorrichtung zum Injizieren einer Substanz nach Anspruch 3,
**dadurch gekennzeichnet, dass** das Antriebselement ein Kurbel-/Pleuelsystem (14) ist.

5. Tragbare Vorrichtung zum Injizieren einer Substanz nach Anspruch 3 oder 4, **dadurch gekennzeichnet, dass** die Injektionsnadel (11) durch den Einspeisekreislauf getragen wird, das Antriebselement mit dem Einspeisekreislauf so verbunden ist, dass, wenn das Antriebselement durch den Servomotor (13) bewegt wird, der Einspeisekreislauf im Injektionsvorrichtungskörper derart bewegt wird, dass die Nadel zwischen ihrer ersten und zweiten Position in Bewegung versetzt wird.

6. Tragbare Vorrichtung zum Injizieren einer Substanz nach Anspruch 5,
**dadurch gekennzeichnet, dass** das Antriebselement fest mit dem ersten Motor (12) verbunden ist.

7. Tragbare Vorrichtung zum Injizieren einer Substanz nach Anspruch 3 oder 4, **dadurch gekennzeichnet, dass** die Injektionsnadel (11) von einem von dem Einspeisekreislauf getrennten Nadelhalter getragen wird, wobei das Antriebselement mit dem Nadelhalter zum Bewegen der Nadel zwischen ihrer ersten und zweiten Position verbunden ist.

8. Tragbare Vorrichtung zum Injizieren einer Substanz nach einem der vorstehenden Ansprüche, **dadurch gekennzeichnet, dass** sie einen Handgriff (16) umfasst.

9. Tragbare Vorrichtung zum Injizieren einer Substanz nach einem der vorstehenden Ansprüche, **dadurch gekennzeichnet, dass** mindestens ein Abschnitt des Einspeisekreislaufs, insbesondere dessen Kammer, in der tragbaren Injektionsvorrichtung nicht horizontal angeordnet ist, wenn der Hauptkörperteil parallel zur Oberfläche des Bodens gehalten wird.

10. Tragbare Vorrichtung zum Injizieren einer Substanz nach einem der vorstehenden Ansprüche, **dadurch gekennzeichnet, dass** sie ein elektronisches Modul umfasst, das zum Anpassen des Einspeisesignals der Spule des ersten Motors in Abhängigkeit vom Widerstandsmoment, das auf den Rotor dieses ersten Elektromotors (12) wirkt, konfiguriert ist.

11. Tragbare Vorrichtung zum Injizieren einer Substanz nach einem der vorstehenden Ansprüche, **dadurch gekennzeichnet, dass** die Kammer zum Festlegen einer Injektionsdosis bemessen ist.

12. Tragbare Vorrichtung zum Injizieren einer Substanz nach einem der vorstehenden Ansprüche, **dadurch gekennzeichnet, dass** sie ein oder mehrere Elemente umfasst, die aus einem Bewegungssensor, einem Temperatursensor, einem Feuchtigkeitssensor, einem Zeitsensor, einem Positionssensor, einer berührungslosen Kommunikationsvorrichtung und/oder einem drahtlosen Kommunikationsmodul zur Kommunikation mit einem Netzwerk ausgewählt sind.

13. Verfahren zur Handhabung einer Vorrichtung zum Injizieren einer Substanz, umfassend einen Einspeisekreislauf mit einer Kammer, die ein erstes Ende mit einer Austrittsöffnung und ein zweites offenes Ende umfasst, einen Kolben, der sich von diesem zweiten Ende aus zum Inneren der Kammer erstreckt, wobei die Kammer aufgrund der Bewegung des Kolbens ein variables Volumen aufweist, einen ersten Elektromotor (12) zum Sicherstellen der Bewegung des Kolbens, wobei die Austrittsöffnung der Kammer zum Einspeisen des Inhalts der Kammer in diese Nadel mit einer zurückziehbaren Injektionsnadel (11) verbunden ist, wobei der Injektionsvorrichtungskörper ein Antriebssystem zum Antreiben der Nadel (11) zwischen einer ersten Position, in der die Nadel innerhalb des Injektionsvorrichtungskörpers aufgenommen ist, und einer zweiten Position, in der die Nadel (11) aus dem Injektionsvorrichtungskörper herausragt, umfasst, wobei das Antriebssystem der Injektionsnadel (11) einen zweiten Motor (13) umfasst, der sich derart von dem ersten Elektromotor (12) unterscheidet, dass die Funktionen des Eintritts/Austritts der Injektionsnadel (11) und des Wiederauffüllens/Abgebens oder des Nachfüllens/Entleerens einer Substanzdosis voneinander getrennt sind, **dadurch gekennzeichnet, dass** die Kammer während der Bewegung der Injektionsnadel (11) aus ihrer zweiten Position in ihre erste Position mit der Substanz gefüllt wird.

14. Verfahren zur Handhabung einer Vorrichtung zum Injizieren einer Substanz nach Anspruch 13, **dadurch gekennzeichnet, dass** die Kammer vollständig mit der Substanz gefüllt wird, um spätestens dann, wenn die Injektionsnadel (11) ihre erste Position erreicht, eine zu injizierende Dosis festzulegen.

## Claims

1. Portable device for injecting a substance, the device comprising a supply circuit which includes a chamber comprising a first end with an outlet and a second open end, a piston extending into said chamber from this second end, said chamber having a volume that can be varied by the movement of the piston, a first electric motor (12) for moving said piston, said outlet of said chamber being connected to a retractable injection needle (11) in order to supply said needle with the contents of said chamber, said injection device including an injection device body, said supply circuit is located in said portable injection device body, the injection device body comprising a system for driving said needle (11) between a first position, in which the needle is housed inside the injection device body, and a second position, in which the needle (11) protrudes from the injection device body, said system for driving said injection needle (11) comprising a second motor (13) that is separate from said first electric motor (12) so that the functions of retracting/deploying the injection needle (11) and replenishing/delivering, or refilling/discharging, a dose of substance are dissociated, and
**characterized in that** said injection device includes means for synchronizing the first electric motor and the second electric motor so that the retraction of the needle after injection of said substance and the replenishing of the chamber are performed simultaneously.

2. Portable substance-injection device according to claim 1,
**characterized in that** the first electric motor (12) is a stepper motor.

3. Portable substance-injection device according to claim 1 or 2,
**characterized in that** said drive system of said injection needle (11) comprises a drive member moved by a servomotor (13).

4. Portable substance-injection device according to claim 3,
**characterized in that** said drive member is a connecting rod/crank system (14).

5. Portable substance-injection device according to claim 3 or 4,
**characterized in that** said injection needle (11) being supported by said supply circuit, said drive member is connected to said supply circuit so that, when said drive member is moved by said servomotor (13), said supply circuit is moved in the injection device body in order to move said needle between its first position and its second position.

6. Portable substance-injection device according to claim 5,
**characterized in that** said drive member is rigidly connected to said first motor (12).

7. Portable substance-injection device according to claim 3 or 4,
**characterized in that** said injection needle (11) being supported by a needle support which is separate from said supply circuit, said drive member is connected to said needle support in order to move said needle between its first position and its second position.

8. Portable substance-injection device according to any one of the preceding claims, **characterized in that** it comprises a gripping handle (16).

9. Portable substance-injection device according to any one of the preceding claims, **characterized in that** at least a portion of the supply circuit, and in particular its chamber, is arranged non-horizontally in the portable injection device when the main body part is held parallel to the floor surface.

10. Portable substance-injection device according to any one of the preceding claims, **characterized in that** it comprises an electronic module configured to adjust the supply signal of the coil of the first motor based on the resisting torque that is being applied to the rotor of this first electric motor (12).

11. Portable substance-injection device according to any one of the preceding claims, **characterized in that** said chamber is sized to determine an injection dose.

12. Portable substance-injection device according to any one of the preceding claims, **characterized in that** it comprises one or more elements selected from among a motion sensor, a temperature sensor, a humidity sensor, a time sensor, a position sensor, a contactless communication device and/or a wireless communication module for communicating with a network.

13. Method for managing a substance-injection device comprising a supply circuit including a chamber comprising a first end with an outlet and a second open end, a piston extending into said chamber from said second end, said chamber having a volume that can be varied by the movement of the piston, a first electric motor (12) for moving said piston, said outlet of said chamber being connected to a retractable injection needle (11) in order to supply said needle with the contents of said chamber, the injection device body comprising a system for driving said needle (11) between a first position, in which the needle is housed inside the body of the injection device, and a second position, in which the needle (11) projects from the body of the injection device, said drive system for said injection needle (11) comprising a second motor (13) that is separate from said first electric motor (12) so that the functions of retracting/deploying the injection needle (11) and replenishing/delivering, or refilling/discharging, a dose of substance are dissociated, **characterized in that** said chamber is filled with said substance during the movement of said injection needle (11) from its second position to its first position.

14. Method for managing a substance-injection device according to claim 13, **characterized in that** said chamber is completely filled with said substance in order to determine a dose to be injected no later than when said injection needle (11) arrives in its first position.
